# EUROPEAN PATENT SPECIFICATION

(11) **EP 0 790 233 B1**
(45) Date of publication and mention of the grant of the patent: **31.10.2001**
(21) Application number: 97102234.8
(22) Date of filing: 12.02.1997
(51) Int. Cl.: C07C 241/02, C07C 243/22

(54) **Method of producing 2-hydrazinonaphtalene compound**
Verfahren zur Herstellung von 2-Hydrazinonaphthalin-Verbindungen
Procédé de préparation de composés de 2-hydrazinonaphthalène

(30) Priority: 16.02.1996 JP 2897796
(43) Date of publication of application: 20.08.1997
(73) Proprietor: FUJI PHOTO FILM CO., LTD., Kanagawa-ken (JP)
(72) Inventor: Yamakawa, Katsuyoshi, Minami-Ashigara-shi, Kanagawa (JP); Sato, Tadahisa, Minami-Ashigara-shi, Kanagawa (JP)
(74) Representative: Grünecker, Kinkeldey, Stockmair & Schwanhäusser Anwaltssozietät

(56) References cited:
- WO-A-95/07888
- GB-A- 934 428

## Description

The present invention relates to a method of producing a 2-hydrazinonaphthalene compound which can be used as an intermediate in synthesizing dyes.

A 2-hydrazinonaphthalene compound is a very important synthesis intermediate, because 1,1-disubstituted-1H-benzo[e]indoles, which are important intermediates of dyes, are derived therefrom in a high yield according to Fischer's indole synthesis, as disclosed in JP-A-3-272890 (The term "JP-A" as used herein means an "unexamined published Japanese patent application") and WO95-07888.

As synthesis methods of a 2-hydrazinonaphthalene compound, the following methods (1), (2) and (3) are known, but each of them has a knotty problem as mentioned below:
(1) the method illustrated below, in which 2-naphthylamine is diazotized and then reduced (Ann. 232, 242 (1886); JP-A-3-272890), 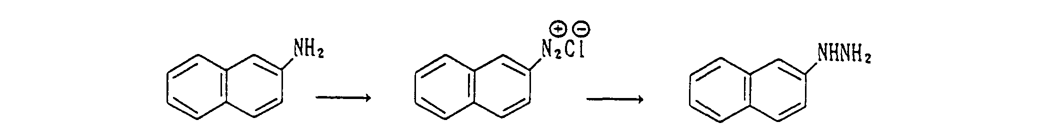
(2) the method illustrated below, in which 2-naphthol is reacted with hydrazine in an autoclave (under high pressure) (J. Prakt, Chem., 78, 143 (1908); WO95-07888), 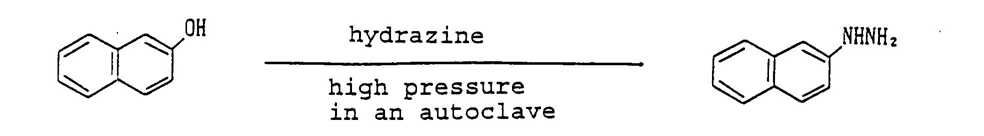
(3) the method illustrated below, in which sodium hydrazide is used (Angew. Chem. Int. Ed. Engl., 6, 84 (1967)),

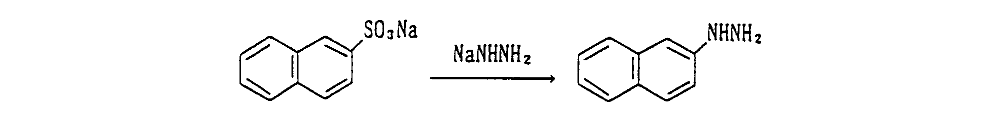

More specifically, the method (1) uses carcinogenic 2-naphthylamine as a starting material, so that such a compound is handled under severe restrictions to cause a considerable increase in production cost.

As for the method (2), an autoclave is required. Although it depends on the material of an autoclave used, the fear of explosion due to the presence of various metal ions and hydrazine is incidental to the method (2).

With respect to the method (3), it is comparatively easy to adopt this method on the scale of a laboratory, but sodium hydrazide is known to explode at a temperature of not lower than 100°C (Angew. Chem. Int. Ed. Engl., 3, 342 (1964). Therefore, it is difficult to use that salt in a large amount.
GB-A-934428 discloses a method for producing a 2-hydrazinonaphthalene derivative by reacting a 2-naphthol derivative with a hydrazine derivative in the presence of sodium hydrogen sulfite.

The present invention aims to provide a method of producing a 2-hydrazinonaphthalene compound at a low price and in safety, which can supplant conventional methods wherein an explosive or carcinogenic compound is employed.

As a result of the inventors' intensive study to solve the problem described above, it has been found that the production method of the present invention can solve the problem.

The present invention provides a method of producing a 2-hydrazinonaphthalene compound represented by the following formula (III), said method comprising the step of reacting a 2-naphthol compound represented by the following formula (I) with a hydrazine compound represented by the following formula (II) in the presence of an acid catalyst: 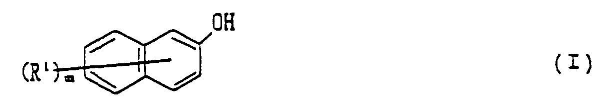 wherein R¹ represents a group by which a naphthalene ring can be substituted, m represents 0 or an integer of from 1 to 7 and, when m is 2 or more, R¹ groups are identical to or different from one another; 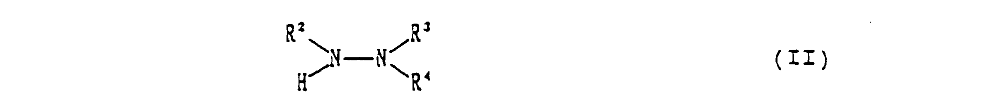 wherein R² represents a hydrogen atom, an alkyl group or an aryl group, and R³ and R⁴ each represents a hydrogen atom, an alkyl group or an aryl group, or R³ and R⁴ complete a ring by combining with each other, or R³ and R⁴ form an alkylidene group in association with each other; 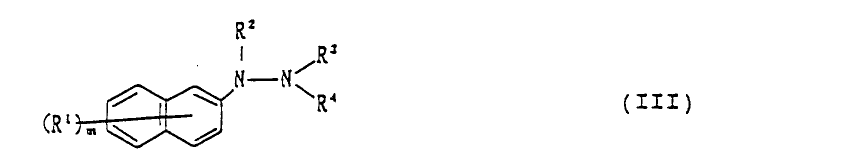 wherein R¹ and m have the same meanings as those in formula (I) respectively, and R², R³ and R⁴ have the same meanings as those in formula (II) respectively.
Preferred embodiments of the present invention are set forth in the claims.

The production method of the present invention is described below in detail.

First, the compounds for use in the present invention are illustrated.

R¹ in formulae (I) and (III) represents a group by which a naphthalene ring can be substituted, with specific examples including a halogen atom (e.g., fluorine, chlorine, bromine, iodine), an alkyl group (e.g., methyl, tert-butyl), an aryl group (e.g., phenyl, naphthyl), a carboxyl group, a cyano group, a carbamoyl group having 1 to 30 carbon atoms, preferably 1 to 15 carbon atoms (e.g., methylcarbamoyl, phenylcarbamoyl), a nitro group, an amino group (e.g., amino, N-methylamino, N,N-dimethylamino), an azo group having 1 to 30 carbon atoms, preferably 1 to 15 carbon atoms (e.g., phenylazo, naphthylazo), an acylamino group having 1 to 30 carbon atoms, preferably 1 to 15 carbon atoms (e.g., acetamido), a sulfonamido group having 1 to 30 carbon atoms, preferably 1 to 15 carbon atoms (e.g., methanesulfonamido), an ureido group, a hydroxy group, an alkoxy group (e.g., methoxy), an aryloxy group (e.g., phenoxy), a sulfo group and a sulfamoyl group having 0 to 30 carbon atoms, preferably 0 to 15 carbon atoms (e.g., methylsulfamoyl, phenylsulfamoyl).

The alkyl group (residue) and the aryl group (residue) represented by the foregoing R¹ and those represented by R², R³ and R⁴, which are described below in detail, are each substituted with another substituent group. As for such a substituent group, the groups as recited above can be examples thereof. The preferable number of carbon atoms contained in the alkyl group, including substituent(s) attached thereto, is from 1 to 20, and the preferable number of carbon atoms contained in the aryl group, including substituent(s) attached thereto, is from 6 to 26.

m represents 0 or an integer of from 1 to 7, preferably 0.

R² in formulae (II) and (III) represents a hydrogen atom, an alkyl group (e.g., methyl) or an aryl group (e.g., phenyl). Preferably, R² is a hydrogen atom.

R³ and R⁴ each represents a hydrogen atom, an alkyl group (e.g., methyl) or an aryl group (e.g., phenyl, naphthyl), or R³ and R⁴ combine with each other to complete a 3- to 7-membered ring. As examples of a 3- to 7-membered ring formed by combining R³ and R⁴, mention may be made of an aziridine ring, a pyrrolidine ring, a piperidine ring, a morpholine ring and a perhydroxyazepine ring. Further, R³ and R⁴ can form an alkylidene group (e.g., 2-propylidene, 3-methylbutyl-2-ylidene) in association with each other.

As for the combination of R², R³ and R⁴, the case where each is a hydrogen atom is preferred in particular.

It is preferable to use a compound represented by formula (II) in an amount of from 0.5 to 50 moles per mole of a compound represented by formula (I). More preferably, the amount of a compound represented by formula (II) used is from 0.8 to 10 moles per mole of a compound represented by formula (I).

The acid used as catalyst in the present invention may be either a proton-donating acid or a Lewis acid.

Specific examples of a proton-donating acid usable as the catalyst include hydrochloric acid, sulfuric acid, phosphoric acid, an organic sulfonic acid (e.g., methanesulfonic acid, trifluoromethanesulfonic acid, benzenesulfonic acid, p-toluenesulfonic acid) and an organic carboxylic acid (e.g., acetic acid, benzoic acid).

Examples of a Lewis acid usable as the catalyst include halides, alkoxides, and salts, such as trifluoromethanesulfonates and perchlorates, of boron, aluminum, titanium, tin, silicon, copper, zinc and lanthanide metals.

The preferable catalyst for the reaction according to the present invention is a proton-donating acid. Especially, sulfuric acid, hydrochloric acid, methanesulfonic acid, trifluoromethanesulfonic acid, benzenesulfonic acid and p-toluenesulfonic acid are used to advantage.

It is preferable for such an acid as recited above to be used in an amount of from 0.05 to 10 moles, particularly from 0.1 to 5 moles, per mole of a compound represented by formula (I).

Examples of a solvent for use in the present invention include aromatic hydrocarbon solvents (e.g., toluene, xylene, mesitylene), alcohol solvents (e.g., ethanol, ethylene glycol), halogen-containing solvents (e.g., chlorobenzene, dichloromethane), organic carboxylic acids (e.g., acetic acid, propionic acid), amide solvents (e.g., N-methylpyrrolidone, 1,3-dimethylimidazolidinone), and water. The preferable amount: of a solvent used is from 50 times to equivalent to, especially from 10 times to equivalent to, the weight of a compound represented by formula (I) used.

In cases where aromatic hydrocarbon solvents are employed, the reaction can also be performed while the water produced is removed by azeotropic distillation using Dean-Stark.

The preferable reaction temperature is from 80°C to 200°C, particularly preferably from 100°C to 150°C, and the preferable reaction time is from 1 to 30 hours, preferably from 2 to 10 hours. The reaction according to the present invention can be carried out at ordinary temperature.

Typical examples of the compounds for use in the present invention are illustrated below.

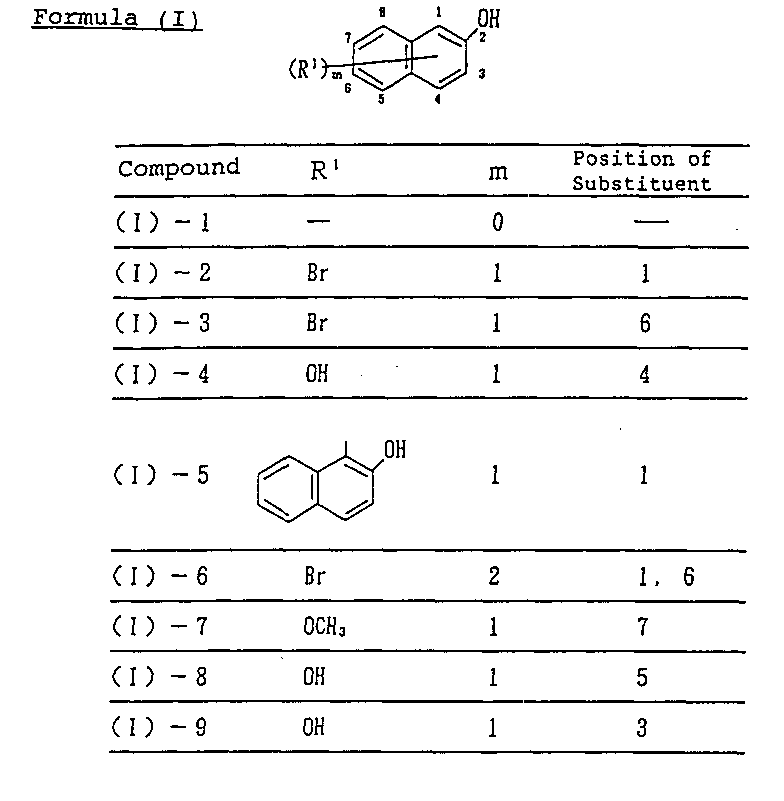

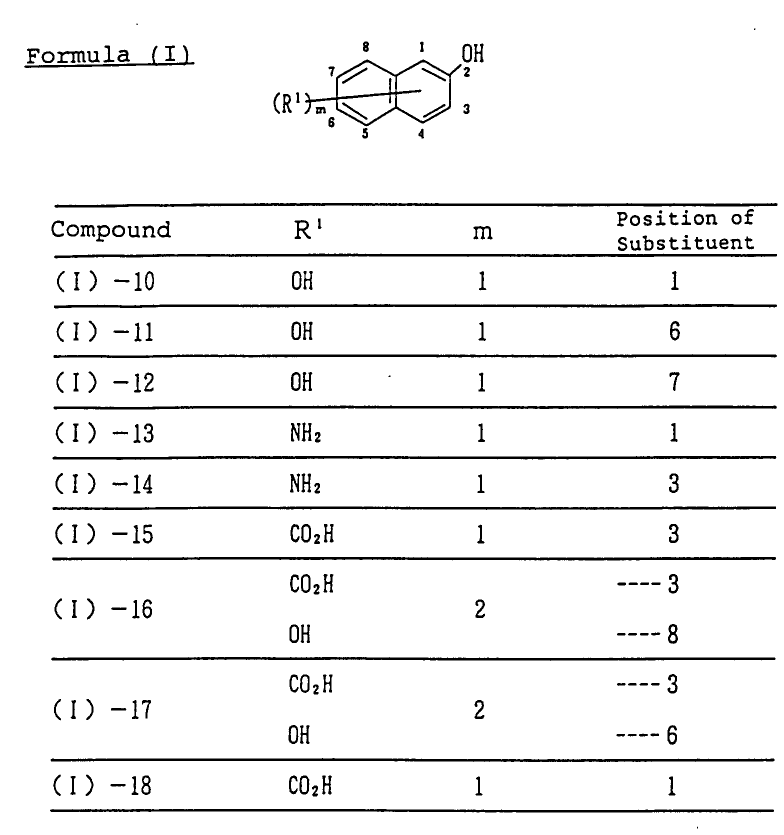

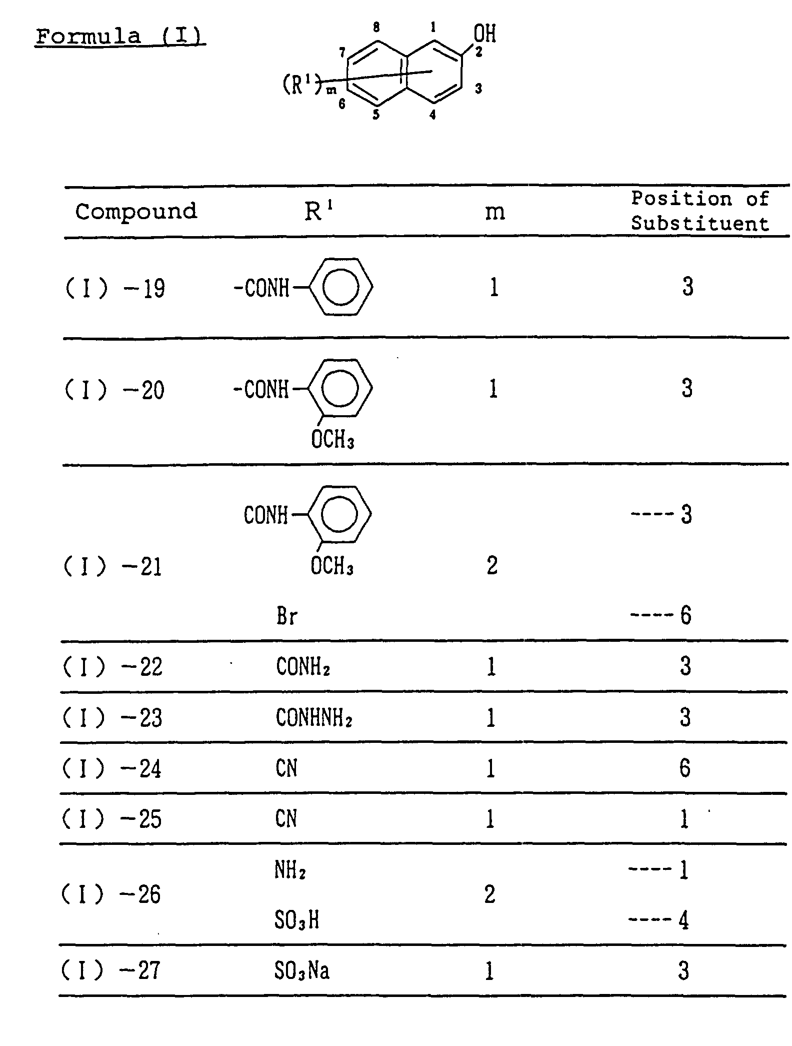

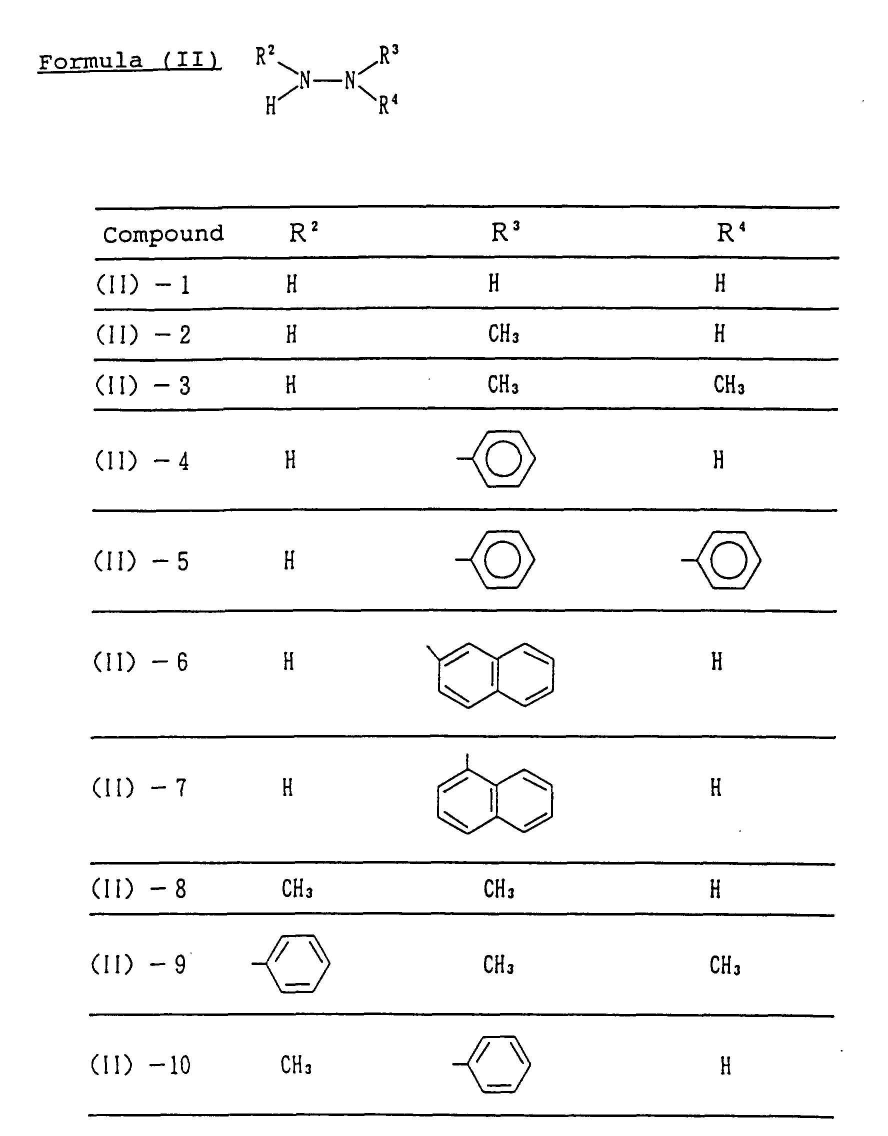

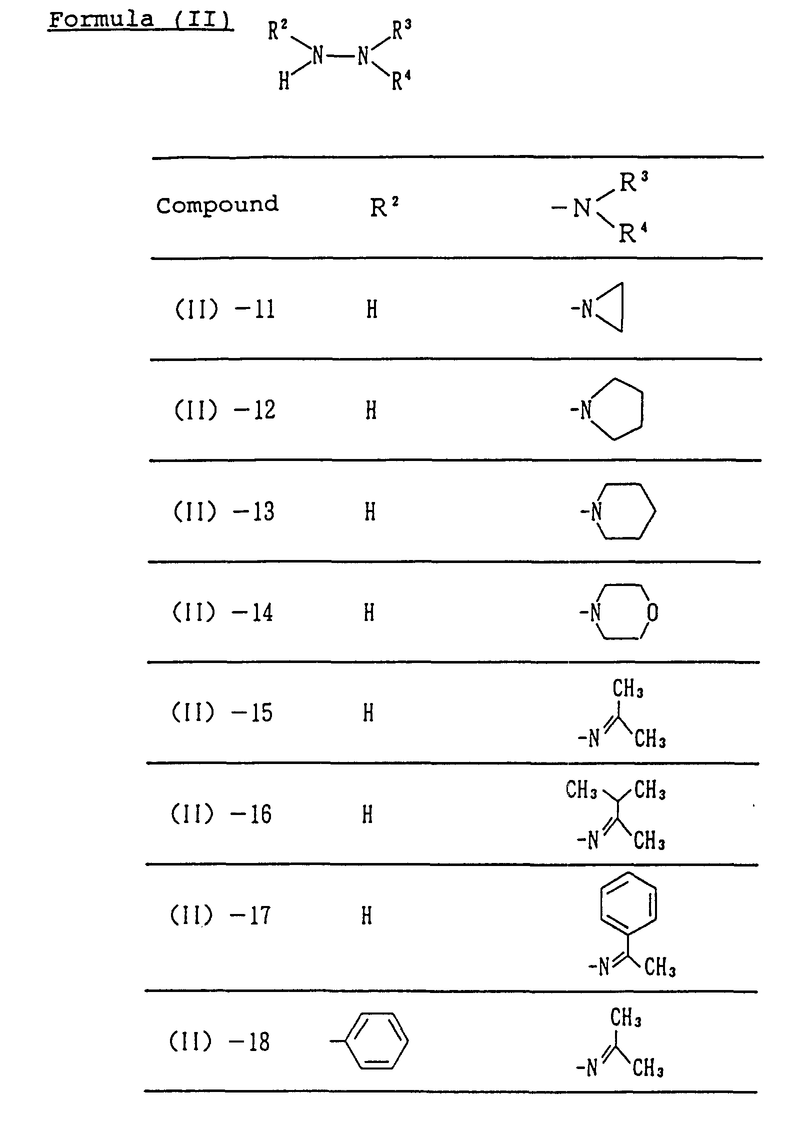

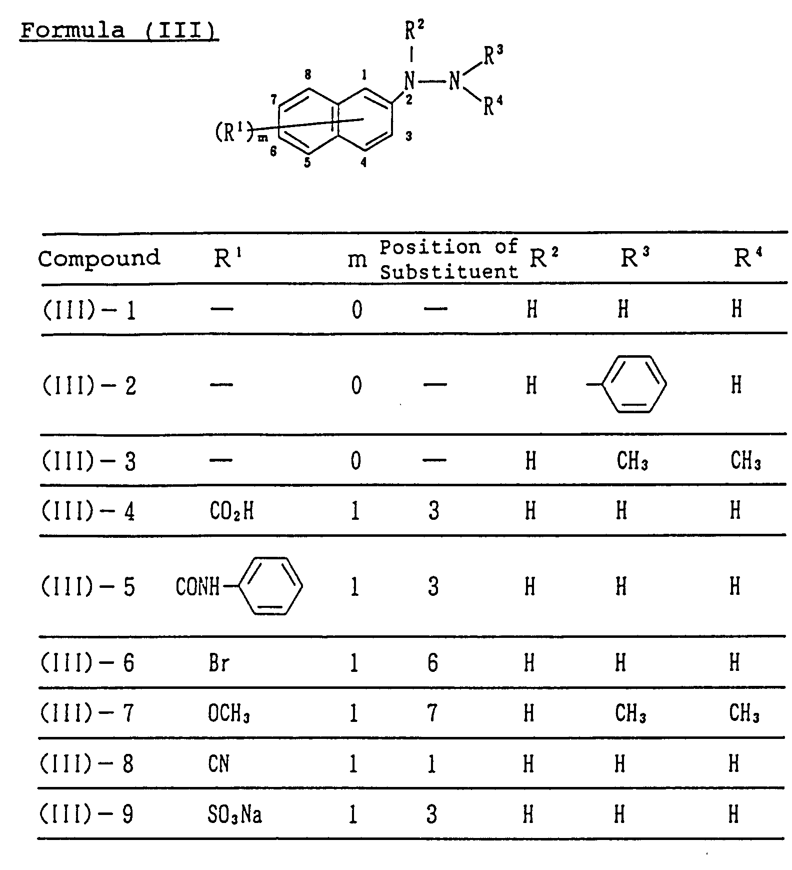

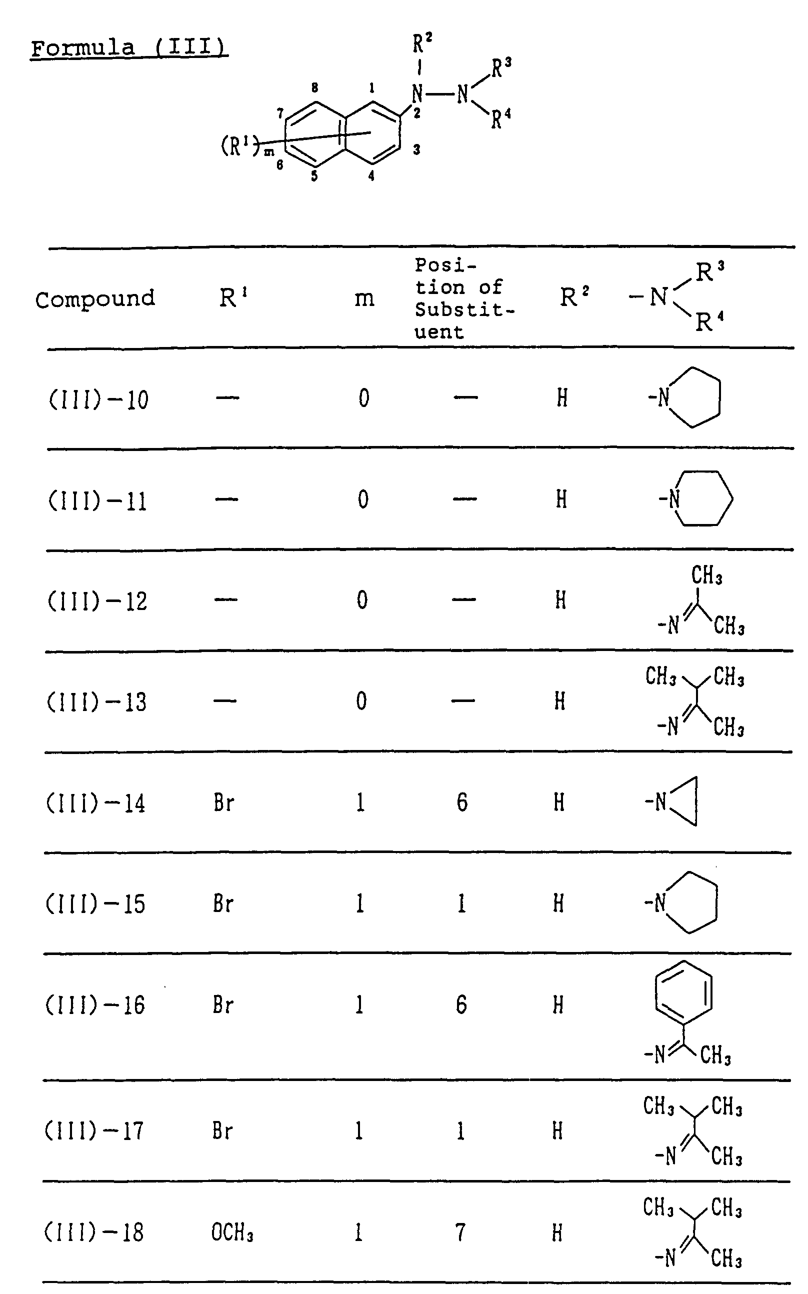

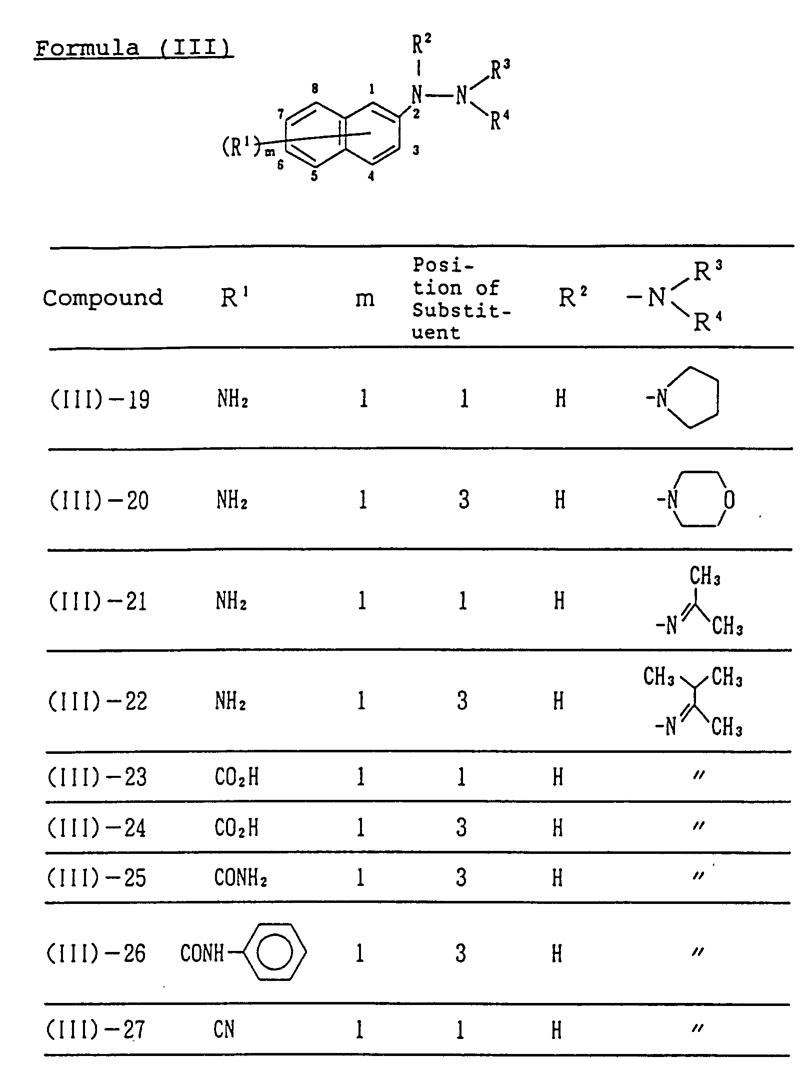

The present invention will now be illustrated in more detail by reference to the following examples:

### EXAMPLE 1

### Synthesis of Compound (III)-1 by the use of Compound (I)-1 and Compound (II)-1:

Compound (I) 1 in an amount of 1.44 g (0.01 mole) and p-toluenesulfonic acid in an amount of 0.57 g (0.013 mole) were dispersed into 20 ml of xylene, and thereto was added 2.0 ml (0.04 mole) of hydrazine hydrate. This dispersion was heated under reflux for 15 hours in an atmosphere of nitrogen. After cooling to room temperature, the reaction mixture was admixed with 20 ml of ethyl acetate and 20 ml of water to separate into solutions. To the residue obtained after the organic solvents were distilled away, 20 ml of hexane and 1.5 ml of concentrated hydrochloric acid were added. The thus precipitated crystals were filtered off to give 1.01 g of Compound (III)-1 in the form of hydrochloride in a 52% yield.

Separately, a reference sample was prepared by converting 2-naphthylamine to the corresponding diazonium salt, and then reducing the diazonium salt with tin(II) chloride.

The spectral analysis data of Compound (III)-1 obtained in accordance with the method of the present invention were in complete agreement with those of the reference sample obtained above.
¹H-NMR (300 MHz: DMSO-d₆)
   - δ ppm: 7.21 (d, 1H, J = 9.0 Hz)
   7.26 (s, 1H)
   7.36 (dd, 1H, J = 8.3, 9.0 Hz)
   7.47 (dd, 1H, J = 8.3, 9.0 Hz)
   7.70 (d, 1H, J = 9.0 Hz)
   7.80 - 7.90 (m, 2H)
   8.61 (s, 1H)
   10.40 (s, 3H)
MS (FAB(posi))

- M + H :: 159 (MW. 158)
142

The other compounds represented by formula (III) were able to be obtained in almost the same manners as described above.

### EXAMPLE 2

### Synthesis of Compound (III)-1 (differing in condition from Example 1):

The same reaction as in Example 1 was carried out, except that the xylene used as solvent was replaced by the same volume of toluene, and the same post-treatment as in Example 1 was performed. Thus, 0.20 g of Compound (III)-1 in the form of hydrochloride was obtained in a 10 % yield.

### EXAMPLE 3

### Synthesis of Compound (III)-1 (differing in condition from Example 1):

The same reaction as in Example 1 was carried out, except that the p-toluenesulfonic acid used as acid catalyst was replaced by methanesulfonic acid in the equimolar amount, and the same post-treatment as in Example 1 was performed. Thus, 1.15 g of Compound (III)-1 in the form of hydrochloride was obtained in a 57 % yield.

### EXAMPLE 4

### Synthesis of Compound (III)-1 (differing in condition from Example 1):

The same reaction as in Example 1 was carried out, except that the xylene used as solvent was replaced by the same volume of ethylene glycol, and the same post-treatment as in Example 1 was performed. Thus, 0.58 g of Compound (III)-1 in the form of hydrochloride was obtained in a 29 % yield.

### COMPARATIVE EXAMPLE 1

### Attempt-1 to synthesize Compound (III)-1 without using acid:

The same reaction as in Example 1 was carried out, except that the p-toluenesulfonic acid as acid catalyst was not used, but the intended Compound (III)-1 was not obtained at all.

### COMPARATIVE EXAMPLE 2

### Attempt-2 to synthesize Compound (III)-1 without using acid:

The same reactants as used in Comparative Example 1 were made to react with each other for 15 hours in triethylene glycol substituted for xylene in the same volume as the temperature of the oil bath was maintained at 200°C during the reaction. After cooling to room temperature, the reaction mixture was admixed with 30 ml of ethyl acetate and 30 ml of water to separate into solutions. After the organic solvents was distilled away, the residue obtained was purified by column chromatography on silica gel. Thus, 0.04 g of Compound (III)-1 in the form of hydrochloride was obtained by mixing the isolated product with hydrochloric acid in n-hexane. The yield of the intended compound was 2 %.

In addition to the intended compound, the above reaction system gave the compound produced by condensation of 2-naphthol and triethylene glycol, and 2,2'-binaphthol produced by the oxidative coupling of 2-naphthol.

This result demonstrates that, in the absence of an acid catalyst, a very high temperature was required to bring about the reaction, and thereby a variety of side-reactions were caused.

Thus, it was proved that the reaction is promoted by the addition of an acid.

Conducting the reaction in the presence of an acid, particularly a proton-donating acid, as shown in Examples, made it possible to render the reaction condition considerably mild, and thereby enabled the economical, safe syntheses of a 2-hydrazinonaphthalene compound.

## Claims

1. A method of producing a 2-hydrazinonaphthalene compound represented by the following formula (III), said method comprising the step of reacting a 2-naphthol compound represented by the following formula (I) with a hydrazine compound represented by the following formula (II) in the presence of an acid catalyst: 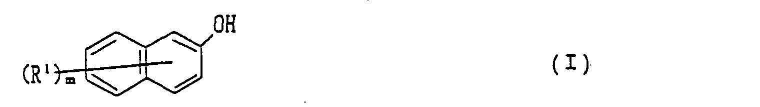 wherein R¹ represents a group by which a naphthalene ring can be substituted, m represents 0 or an integer of from 1 to 7 and, when m is 2 or more, R¹ groups are identical to or different from one another; 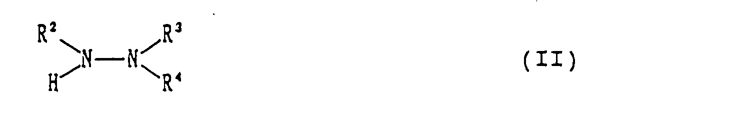 wherein R² represents a hydrogen atom, an alkyl group or an aryl group, and R³ and R⁴ each represents a hydrogen atom, an alkyl group or an aryl group, or R³ and R⁴ complete a ring by combining with each other, or R³ and R⁴ form an alkylidene group in association with each other; 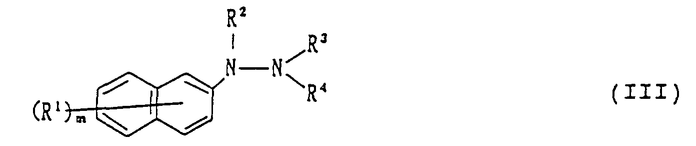 wherein R¹ and m have the same meanings as those in formula (I) respectively, and R², R³ and R⁴ have the same meanings as those in formula (II) respectively.

2. The method as claimed in Claim 1, wherein the compound represented by formula (II) is used in an amount of from 0.5 to 50 moles per mole of the compound represented by formula (I).

3. The method as claimed in Claim 2, wherein the compound represented by formula (II) is used in an amount of from 0.8 to 10 moles per mole of the compound represented by formula (I).

4. The method as claimed in Claim 1, wherein the acid catalyst is selected from the group consisting of hydrochloric acid, sulfuric acid, phosphoric acid, an organic sulfonic acid, an organic carboxylic acid, and halides, alkoxides, trifluoromethanesulfonates and perchlorates of boron, aluminum, titanium, tin, silicon, copper, zinc and lanthanide metals.

5. The method as claimed in Claim 4, wherein said organic sulfonic acid is selected from the group consisting of methanesulfonic acid, trifluoromethanesulfonic acid, benzenesulfonic acid and p-toluenesulfonic acid.

6. The method as claimed in Claim 1, wherein the acid catalyst is used in an amount of from 0.05 to 10 moles per mole of the compound represented by formula (I).

7. The method as claimed in Claim 6, wherein the acid catalyst is used in an amount of from 0.1 to 5 moles per mole of the compound represented by formula (I).

8. The method as claimed in Claim 1, wherein the reaction temperature is from 80°C to 200°C.

9. The method as claimed in Claim 8, wherein the reaction temperature is from 100°C to 150°C.

10. The method as claimed in Claim 1, wherein the reaction time is from 1 to 30 hours.

## Patentansprüche

1. Verfahren zur Herstellung einer 2-Hydrazinonaphthalinverbindung, dargestellt durch die folgende Formel (III), umfassend das Umsetzen einer 2-Naphtholverbindung, dargestellt durch die folgende Formel (I), mit einer Hydrazinverbindung, dargestellt durch die folgende Formel (II), in Gegenwart eines Säurekatalysators: 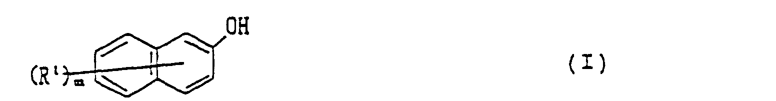 worin R¹ ein Rest ist, der an den Naphthalinring gebunden ist, m ist 0 oder eine ganze Zahl im Bereich von 1 bis 7, und wenn m größer als 1 ist, können die Reste R¹ gleich oder verschieden sein; 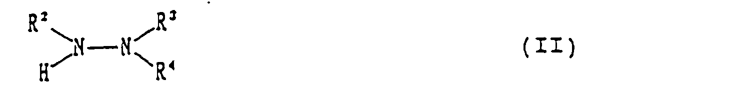 worin R² ein Wasserstoffatom, eine Alkylgruppe oder eine Arylgruppe ist, und R³ und R⁴ bedeuten jeweils ein Wasserstoffatom, eine Alkylgruppe oder eine Arylgruppe, oder R³ und R⁴ können miteinander verbunden sein, um einen Ring zu bilden, oder R³ und R⁴ können zusammen eine Alkylidengruppe bilden; 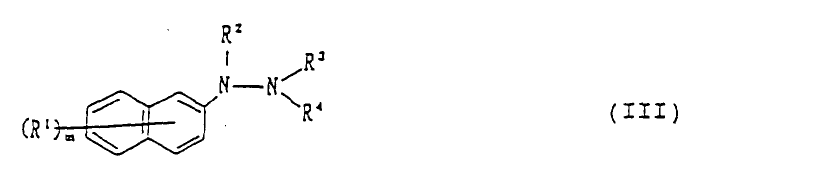 worin R¹ und m jeweils die gleiche Bedeutung wie in Formel (I) haben, und R², R³ und R⁴ haben jeweils die gleiche Bedeutung wie in Formel (II).

2. Verfahren nach Anspruch 1, wobei die Verbindung, die durch die Formel (II) dargestellt ist, in einer Menge im Bereich von 0,5 bis 50 Mol pro Mol der Verbindung, die durch die Formel (I) dargestellt ist, verwendet wird.

3. Verfahren nach Anspruch 2, wobei die Verbindung, die durch die Formel (II) dargestellt ist, in einer Menge im Bereich von 0,8 bis 10 Mol pro Mol der Verbindung, die durch die Formel (I) dargestellt ist, verwendet wird.

4. Verfahren nach Anspruch 1, wobei der Säurekatalysator ausgewählt ist aus der Gruppe, bestehend aus Salzsäure, Schwefelsäure, Phosphorsäure, einer organischen Sulfonsäure, einer organischen Carbonsäure und Halogeniden, Alkoxiden, Trifluormethansulfonaten und Perchloraten von Bor, Aluminium, Titan, Zinn, Silicium, Kupfer, Zink und Metallen der Lanthanreihe.

5. Verfahren nach Anspruch 4, wobei die organische Sulfonsäure ausgewählt ist aus der Gruppe, bestehend aus Methansulfonsäure, Trifluormethansulfonsäure, Benzolsulfonsäure und p-Toluolsulfonsäure.

6. Verfahren nach Anspruch 1, wobei der Säurekatalysator in einer Menge im Bereich von 0,05 bis 10 Mol pro Mol der Verbindung, die durch die Formel (I) dargestellt ist, verwendet wird.

7. Verfahren nach Anspruch 6, wobei der Säurekatalysator in einer Menge im Bereich von 0,1 bis 5 Mol pro Mol der Verbindung, die durch die Formel (I) dargestellt ist, verwendet wird.

8. Verfahren nach Anspruch 1, wobei die Umsetzung bei einer Temperatur im Bereich von 80 °C bis 200 °C durchgeführt wird.

9. Verfahren nach Anspruch 8, wobei die Umsetzung bei einer Temperatur im Bereich von 100 °C bis 150 °C durchgeführt wird.

10. Verfahren nach Anspruch 1, wobei die Umsetzung 1 bis 30 Stunden lang durchgeführt wird.

## Revendications

1. Procédé de préparation d'un composé 2-hydrazinonaphtalène représenté par la formule (III) ci-dessous, ledit procédé comprenant l'étape de réaction d'un composé 2-naphtol représenté par la formule (I) ci-dessous avec un composé hydrazine représenté par la formule (II) ci-dessous en présence d'un catalyseur acide : 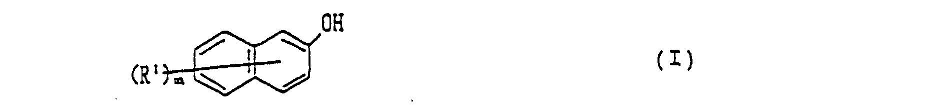 dans laquelle R¹ représente un groupe par lequel un cycle naphtalène peut être substitué, m représente 0 ou un nombre entier de 1 à 7 et, lorsque m est égal ou supérieur à 2, les groupes R¹ sont identiques ou différents l'un de l'autre; 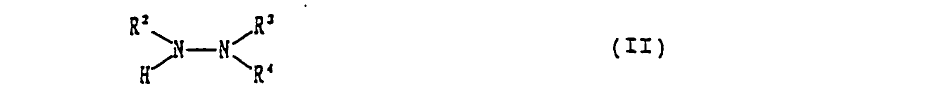 dans laquelle R² représente un atome d'hydrogène, un groupe alkyle ou un groupe aryle et R³ et R⁴ représentent chacun un atome d'hydrogène, un groupe alkyle ou un groupe aryle ou R³ et R⁴ complètent un cycle en se combinant l'un à l'autre ou R³ et R⁴ forment un groupe alkylidène en association l'un avec l'autre ; 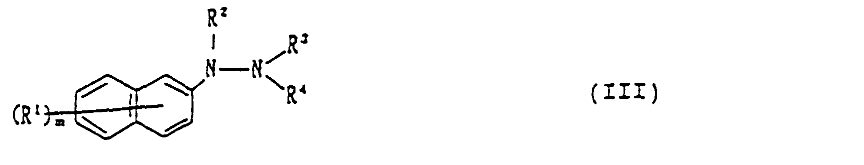 dans laquelle R¹ et m ont les respectivement mêmes significations que celles dans la formule (I) et R², R³ et R⁴ ont les respectivement mêmes significations que celles de la formule (II).

2. Procédé tel que revendiqué dans la revendication 1, dans lequel le composé représenté par la formule (II) est utilisé en une quantité de 0,5 à 50 moles par mole de composé représenté par la formule (I).

3. Procédé tel que revendiqué dans la revendication 2, dans lequel le composé représenté par la formule (II) est utilisé en une quantité de 0,8 à 10 moles par mole de composé représenté par la formule (I).

4. Procédé tel que revendiqué dans la revendication 1, dans lequel le catalyseur acide est choisi parmi le groupe constitué de l'acide chlorhydrique, l'acide sulfurique, l'acide phosphorique, un acide sulfonique organique, un acide carboxylique organique, et des halogénures, des alcoxydes, des trifluorométhanesulfonates et des perchlorates du bore, de l'aluminium, du titane, de l'étain, du silicium, du cuivre, du zinc et des métaux lanthanidiques.

5. Procédé tel que revendiqué dans la revendication 4, dans lequel ledit acide sulfonique organique est choisi parmi le groupe constitué de l'acide méthanesulfonique, l'acide trifluorométhanesulfonique, l'acide benzènesulfonique et l'acide p-toluènesulfonique.

6. Procédé tel que revendiqué dans la revendication 1, dans lequel le catalyseur acide est utilisé en une quantité de 0,05 à 10 moles par mole de composé représenté par la formule (I).

7. Procédé tel que revendiqué dans la revendication 6, dans lequel le catalyseur acide est utilisé en une quantité de 0,1 à 5 moles par mole de composé représenté par la formule (I).

8. Procédé tel que revendiqué dans la revendication 1, dans lequel la température de réaction est de 80 °C à 200 °C.

9. Procédé tel que revendiqué dans la revendication 8, dans lequel la température de réaction est de 100 °C à 150 °C.

10. Procédé tel que revendiqué dans la revendication 1, dans lequel la durée de réaction est de 1 à 30 heures.
